Europäisches Patentamt

(19) European Patent Office

Office européen des brevets

(11) Publication number: **0 186 845**
**B1**

(12) **EUROPEAN PATENT SPECIFICATION**

(45) Date of publication of patent specification: **23.05.90**

(21) Application number: **85116049.9**

(22) Date of filing: **16.12.85**

(51) Int. Cl.⁵: **G 01 N 30/88, G 01 N 30/86**

(54) A method and equipment for the simulated distillation by means of gas chromatography with non vaporizing direct injection.

(30) Priority: **28.12.84 IT 2427884**

(43) Date of publication of application:
**09.07.86 Bulletin 86/28**

(45) Publication of the grant of the patent:
**23.05.90 Bulletin 90/21**

(84) Designated Contracting States:
**AT BE CH DE FR GB LI NL SE**

(56) References cited:
**US-A-3 169 389**

**JOURNAL OF CHROMATOGRAPHIC SCIENCE, vol. 14, February 1976, pages 49-51; B.W. JACKSON et al.: "Boiling Range Distribution of Petroleum with a Short Capillary Column"**

**ANALYTICAL CHEMISTRY, vol. 42, no. 12, October 1970, pages 1394-1399; T.H. GOUW et al.: "Versatile Short Capillary Column in Gas Chromatography"**

(73) Proprietor: **CARLO ERBA STRUMENTAZIONE S.p.A.**
**I-20090 Rodano Milano (IT)**

(72) Inventor: **Trestianu, Sorin**
**15, Avenue Mercure**
**Bruxelles (BE)**
Inventor: **Munari, Fausto**
**59, Corso Lodi**
**Milan (IT)**
Inventor: **Saravalle, Carlo**
**29, Via Marostica**
**Milan (IT)**

(74) Representative: **Marietti, Giuseppe**
**CENTRO DI CONSULENZA IN PROPRIETA' INDUSTRIALE Viale Caldara, 43**
**I-20122 Milano (IT)**

(56) References cited:
**ANALYTICAL CHEMISTRY, vol. 36, no. 8, July 1964, page 1512-1516; L.E. GREEN et al.: "Simulated Distillation by Gas Chromatography"**

Courier Press, Leamington Spa, England.

(56) References cited:

JOURNAL OF HIGH RESOLUTION
CHROMATOGRAPHY & CHROMATOGRAPHY
COMMUNICATIONS", vol. 8, November 1985,
pages 771-781, S. TRESTIANU: "Automatic
Simulated Distillation of Heavy Petroleum
Fractions up to 800C degrees TBP by Capillary
Gas Chromatography"

JOURNAL OF HIGH RESOLUTION
CHROMATOGRAPHY & CHROMATOGRAPHY
COMMUNICATIONS, vol. 8, April 1985, pages
193-195; L.A. LUKE et al.: "Simulated Distillation
of Atmospheric Residues Using Short Pyrex
Capillary Columns"

Journal of Chromatography, 203 (1981) 193-205,
M. Galli + S. Trestianu "Benefits of a Special
Cooling System in Non-Vaporizing On-Column
Injection Procedures"

## Description

The present invention concerns a method and an equipment for a simulated distillation, by means of gas chromatography, of petroleum fractions, in particular, even if not exclusively, of petroleum heavy residues deriving from atmospheric and vacuum distillation. These residues are assigned to undergo further treatments of cracking, for their industrial conversion into white products.

In order to characterize components, derivatives and residues of petroleum, for any purpose, for instance for choosing the type of their industrial treatment and for the control of relevant processes and plants, a widely used method of analysis is distillation.

Laboratory distillation tests are used for example to determine the boiling temperature range of petroleum products by a distillation curve, for instance a so called Eagler distillation curve, which reports the total percentage of distillate for each temperature value.

However plotting of said curves requires a distillation operation which is extremely long and burdensome and does not allow the characterization of heavy fractions which require very high temperatures for obtaining a product distillation. In fact, conventional distillation, even if carried out under conditions of reduced pressure (ASTM D1160), generally is not suitable for samples with boiling points exceeding 450—500°C (see for example paragraph 10.13 ASTM D2892).

A better characterization of heavy petroleum products is obtained using an instrumentation for short path vacuum distillation, such as for example the DISTACT System (Total CFR/Leybold Hereus) (P. Vercier and M. Mouton—Analusis 10, 1982-57) allowing the obtention of fractions with boiling points exceeding 600°C without significant thermal degradation. However, this type of instrumentation, even if it does not produce significant thermal degradation of the components, is difficulzcto operate for the characterization of products through their boiling point distribution curves. Moreover it does not permit to correlate the boiling points under vacuum with those at atmospheric pressure.

In order to avoid the drawbacks of distillation procedures, it has been already proposed to simulate the distillation up to the actual boiling point by means of a temperature programmed low resolution gas chromatographic analysis, (F. T. Eggerston, S. Groennings and J. J. Holst, Anal. Chem. 32: 904—909 (1960). (See also R. Butter in: Chromatography in Petroleum Analysis, K. Altgelt and T. Gouw Editors, M. Dekker, NY 1979, page 75).

This proposal is based on the fact that hydrocarbons are eluted in a non polar column in the order of their relevant boiling points and it is therefore possible to convert the retention times into distillation temperatures. In this way, using packed columns with non polar stationary phases (generally of polydimethylxyloxane type or of the DEXSIL (Registered Trademark of Dexsil Chemical Corporation—Hamden—Connecticut——USA) type—based on carbonium and silicium), it is possible to obtain a chromatogram representing the sample elution curve, wherein to each retention time corresponds a given boiling point. The retention times are converted into boiling points by means of a boiling points—retention times curve plotted by performing a previous calibration with a known standard sample (generally mixtures of normal-alkanes). Once the time axis is calibrated in temperatures, the surface comprised within the elution curve and the base line of the chromatogram is integrated in slices and summed up, thus obtaining the cumulative surfaces corresponding to each boiling temperature. The graphic display of said cumulative surfaces (in % of the total surface comprised between the elution curve and the base line), as a function of the boiling points, provides a "simulated" distillation boiling point distribution curve (similar to the Engler distillation curve).

The hereinabove described technique is well known and applied and is already standardized (see for example ASTM D 2887 "Boiling Range Distribution of Petroleum Fraction by Gas Chromatography").

However it is also known that this simulated distillation technique, which gives undeniable advantages of time reduction (in order of 1:100) and energy savings in comparison with the conventional distillation, shows some limitations when heavy or high-boiling samples have to be analysed. In fact, because of the limitations in the highest temperature at which the packed column can be heated and due to extremely long retention times of high-boiling compounds at the temperatures of the analytical program, it is generally impossible to characterize hydrocarbons having boiling points exceeding approximately 600°C, that correspond to n-al alkanes with a number of carbon atoms around C55—C60.

Document: Journal of Chromatographic Science vol. 14 February 1976 pages 49 to 51; B. W. Jackson et al.: "Boiling Range Distribution of Petroleum with a short Capillary Column" describes a simulated distillation system in which:

a) the oven is heated to a temperature up to 350°C,

b) the column has a length of 10 m and an inner diameter of 0.254 mm,

c) the injector is a vaporisation injector.

The use of a conventional vaporisation injector introduces a heavy discrimination of high boiling sample components; moreover said components are at least partly pyrolysed in the injector and disturb the analysis. This allows to obtain a quantitative determination of the petroleum products which is necessarily limited to about C30, as the heaviest components remain partly in the vaporization injector.

Document: Journal of Chromatography, *203*

(1981) 193—205; M. Galli and S. Trestianu: "Benefits of a Special Cooling System to Improve Precision and Accuracy in Non-Vaporizing on Column Injection Procedures" describes the benefits of using a cold non-vaporizing on-column injector in gas-chromatographic analyses, comprising avoiding discrimination and destruction of sample heavy components in the injector.

The analyses are carried out, after the injection, in a conventional manner, with an oven programming temperature up to 350°C—360°C.

Utilizing a cold on-column injector according to this document in a simulated distillation apparatus according to the first one, would lead to some improvement of the operating conditions, due to the fact that said pyrolysis and discrimination in the injector do not occur. This improvement would lead to a quantitative analysis of components heavier than those analysed with the first document, with an improvement of this hypothetical combination from C30 to C50—C60.

But if samples having components heavier than C60 are analyzed, they create problems due to the fact that eluting said heavy fractions requires hours and hours and that said elution must be necessarily performed before starting a new analysis.

Accordingly, as happens for in the conventional simulated distillation systems, the heavy part mf the sample is "cut" at about C60 by using a precolumn mounted in front of the analytical column.

The heavy part of the sample is not released to the analytical column but backpurged from the precolumn during the regular elution of the more volatile part from the analytical column.

Document: Analytical Chemistry Vol. 42, N. 12, October 1970, page 1394 to 1399; T. H. Gouw et al.: "Versatile Short Capillary Column in Gas Chromatography" describes the use of a short (10 meters) capillary column having a relatively reduced inner diameter (about 0.254 mm) to analyze heavy petroleum fractions up to C58. However the quantitative determination is only up to C42 and the oven maximum temperature is 360°C.

Stating what precedes, the object of this invention is to provide a method and an equipment for the simulated distillation of petroleum products by gas chromatography, which are capable of performing also the analysis of heavy petroleum products, such as for example residues of distillation under vacuum, containing molecules with a boiling point exceeding 600°C, up to about 800°C TPB, that corresponds to n-alkanes containing a number of carbon atoms exceeding 130.

According to the invention, said object is achieved by a method for the simulated distillation of petroleum products, in particular heavy residues of petroleum, by means of chromatographic analysis and subsequent slice integration of the surface comprised between the elution curve of the sample and the chromatogram base line, with conversion of the retention times into boiling temperatures by means of a standard mixture, of the type comprising the following steps:

a) providing an equipment of gas chromatographic analysis, comprising:

i) an injector for capillary column;

ii) a gas chromatographic capillary column;

iii) an oven, wherein the column is housed, programmable in temperature;

iv) a detector of the flame ionization type;

b) injecting an appropriately diluted sample of a petroleum product;

c) performing a gas chromatographic analysis with a temperature program in the oven;

d) converting the time axis of the obtained chromatogram into boiling point axis by using adequate calibration mixtures which permit the obtention of a standard curve;

e) integrating in slices the surface between the sample elution curve and the chromatogram base line to obtain a simulated distillation boiling point distribution curve, characterized in that:

i) said injector is of the direct non vaporizing cold on-column type;

ii) said cpillary column has an inner diameter ranging between 0.5 and 0.6 mm and a length greater than 1 m and shorter than 10 m, with a thin film stationary phase less than or equal to 0.2 μm, and in that said temperature program in the oven is carried out with a programmation speed equal to at least 5°C/min, up to temperatures higher than 400°C.

As it will be seen later on, it is advisable that the equipment includes a pneumatic system to feed the carrier, which allows said feeding to be performed at a constant pressure during the injection and at a constant flow during the analysis.

Moreover, the injected sample can be previously treated to remove asphaltenes (if present in the same) and diluted to sample concentrations of approximately 10 g/l. Asphaltene removal is carried out by solution in n-heptane, followed by filtration of asphaltenes (for example as described in Standards IP-143/78 or NF-T 60115) and partial evaporation of the solvent, which however still remains as sample solvent.

It is also possible to operate without de-asphaltation, taking into account that the unelutable part of asphaltenes represent "dirts" deposited into the initial section of the column and therefore diminuishing its lifetime. In such cases carbon disulfide is the commonly used solvent. Finally, the conversion of the chromatogram time into boiling temperatures is performed by using a standard containing heavy alkanes beyond C80 or consisting for example of products such as POLYWAX (Registered Trademark of Bareco Division of Petrolite Corporation—Tulsa—Oklahoma—USA) and with extrapolation of the boiling temperature curve in function of the retention times.

The above mentioned method according to the invention, can be carried out by an equipment comprising:

i) an injector for capillary columns;

ii) a gas chromatographic capillary column;

iii) an oven, wherein the column is housed, programmable in temperature;

iv) a detector of the flame ionization type, characterized in that said injector is of direct non vaporizing cold on-column type, that said capillary column has an inner diameter ranging between 0.5 and 0.6 mm and a length greater than 1 m and shorter than 10 m, with a thin layer stationary phase in a thickness lower than or equal to 0.2 µm, and in that said oven is programmable over 400°C with a programmation rate equal to at least 5°C/min.

The above method and equipment allow to attain results never achieved up to now in simulated distillation of petroleum products, with reference to the characterization of high-boiling components, such as compounds with molecules >C55.

These results are obtained by acting both on the oven temperature, which has been raised above 400°C, generally up to approximately 430/450°C and mainly on the features of the column which is a short and wide capillary column.

In fact this allows to achieve the desired results, i.e. a detectable elution of hydrocarbons having carbon number well over C60 without increasing the oven temperatures above the limits presently imposed by the stationary phase; this is obtained by a reduction of the column retention capacity through an action on two factors; namely: the column length and diameter, and the thickness of the stationary phase film.

The length of the capillary column is kept within values of 1 m and 10 m. Below 1 m length there is not sufficient power of separation. Over 10 m the length is too much and therefore the retention time of heavy compounds is too high.

The stationary phase present in the gas chromatographic column can be one of the phases used up to now for simulated distillation (for instance poly/dimethylsilicones) and forms a thin layer having a thickness less than 0.2 µm, for example 0.1 µm. In this way it is possible to reduce the elution time of the high boiling compounds in respect of the known simulated distillations, with possibility to analyze hydrocarbons over C100.

The standard used to convert the retention times must be such as to cover the range of compounds >C55. It is suggested to use for this purpose polyethylenes with average molecular weight from 500 to 1500, for example the compounds known under the Registered Trademark POLYWAX.

It must be noticed that the boiling points of polyethylenes containing more than 60 carbon atoms are calculated by extrapolation of the curve obtained by plotting the boiling points of n-alkanes in function of their number of carbon atoms.

It is necessary that the sample injection into the column takes place without discrimination and in such a manner as not to introduce polluting components. The use of vaporizing conventional injectors with septum is therefore excluded, in that the chromatogram could report also the analysis of the decomposition products from septum fractions which enter the vaporizer. Furthermore, the sample injection into a heated environment to produce vaporization causes the discrimination of high-boiling compounds, which partly remain in the syringe needle or in the heated sampling system.

On the contrary, a non vaporizing direct cold on-column injector has proved to be really appropriate, as said injector ensures that the whole liquid sample enters the column. The only problem experienced with such an injector is the possibility that very heavy compounds remain in the starting part of the column, which compounds are not eluted and can damage the column. The problem is solved by periodically physically eliminating the column section involved or by submitting the same to a de-asphaltation treatment. In these cases, it is advisable that a starting part of the capillary column, for example approximately 1 m long, is empty and deactivated.

The column can be made of glass, metal (for instance Ni) or fused silica, with an inner diameter exceeding 0.2 mm (to allow the needle introduction) and less than 0.6 mm to avoid problems of pressure fall in the column itself and to maintain a certain separatory power of the same.

In case of fused silica columns, which have an external polyimide coating, it must be noticed that said coating oxidizes at temperatures exceeding 380—400°C. Therefore there must be an atmosphere of inert gas in the oven or fused silica columns coated with metal instead of with polyimide must be used.

Due to the use of short columns, it is difficult to control the pressure fall along the column, but in any case such pressure fall remains low. Therefore a non vaporizing direct on-column injector is used with carrier gas fed at a constant pressure during the sample injection stage and at a constant flow in the subsequent stage of chromatographic analysis.

The carrier gas is preferably hydrogen, the optimal flow of which is at least double in respect of all other gases, which allows to furtherly reduce the elution times. However it is possible to use helium as well.

The detector must be of the flame ionization type (FID=Flame Ionization Detector). As it is known, the response of this type of detector strongly varies with the hydrogen flow, which is the preferred carrier gas. The use of hydrogen and of an oven temperature programme up to temperatures exceeding 400°C result in a decrease of hydrogen flow through the column if the carrier gas is fed at a constant pressure. This variation produces a change in the detector response with the oven temperature and therefore introduces an error factor in the results. Therefore, in order to eliminate this drawback, it is necessary to use a pneumatic system allowing a carrier feeding at a constant flow during analysis. Finally, the FID detector body, between the outlet port of the gas chromatographic column from the oven and the detector flame, is heated by suitable means, in order to avoid colder points in respect of the temperature set for the detector and of the maximum programmed temperature for the oven.

In order to show the advantageous features of the invention, the result of an analysis as performed on a sample consisting of a deasphalted residue under vacuum with an initial boiling point at approx. 550°C is hereinafter reported.

The analytical equipment used was the following one:

Gas Chromatographic apparatus type Mega HT of Carlo Erba Strumentazione S.p.A. with a non vaporizing direct on-column injector, type OC50; column with inner diameter 0.32 mm having an empty and deactivated initial section of 1.00 m and a length of the following part, bearing the stationary phase of 9.00 m, said stationary phase, consisting of polydimethylsilicones in a layer of about 0.08 μm thickness.

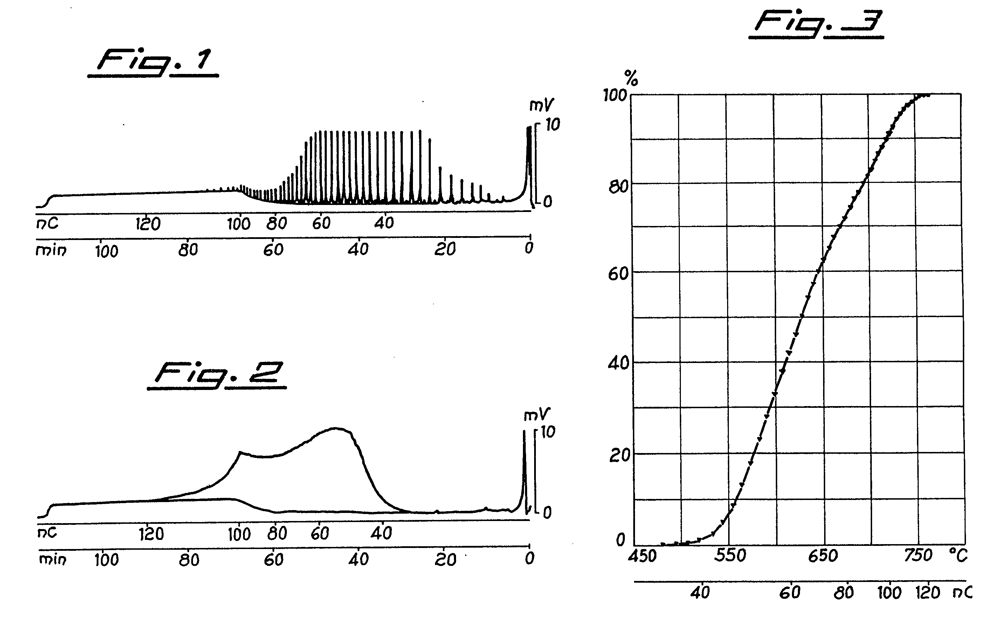

The temperature programme of the oven was performed starting from 60°C up to 430°C with a rate of 5°C/min. The chromatogram reported in Figure 1 was obtained. The curve reported in Figure 2 was obtained by using a standard consisting of Polywax 655. The chromatogram of Figure 1 was then conventionally processed on the basis of the curve of Figure 2 to obtain the simulated distillation curve as reported in Figure 3.

## Claims

1. A method for the simulated distillation of peotrolum products, in particular heavy residues of petroleum, by means of chromatographic analysis and subsequent slice integration of the surface comprised between the elution curve of the sample and the chromatogram base line, with conversion of the retention times into boiling temperatures by means of a standard mixture, of the type comprising the following steps:

a) providing an equipment of gas chromatographic analysis, comprising:

i) an injector for capillary column;

ii) a gas chromatographic capillary column;

iii) an oven wherein the column is housed, programmable in temperature;

iv) a detector of the flame ionization type;

b) injecting an appropriately diluted sample of a petroleum product;

c) performing a gas chromatographic analysis with a temperature program in the oven;

d) converting the time axis of the obtained chromatogram into boiling point axis by using adequate calibration mixtures which permit the obtention of a calibration curve;

e) integrating in slices the surface between the sample elution curve and the chromatogram base line to obtain a simulated distillation boiling point distribution curve, characterized in that:

i) said injector is of the direct non vaporizing cold on-column type;

ii) said capillary column has an inner diameter ranging between 0.5 and 0.6 mm and a length greater than 1 m and shorter than 10 m, with a thin film stationary phase, less than or equal to 0.2 μm, and in that said temperature program in the oven is carried out with a programmation speed equal to at least 5°C/min, up to temperatures higher than 400°C.

2. A method according to claim 1, characterized in that said cold injection and said chromatographic analysis are carried out by feeding the carrier gas at a constant pressure and at a constant flow, respectively.

3. A method according to claim 1, wherein a known substance is used for the definition of said standard curve, consisting of a polyethylene POLYWAX® with average molecular weight from 500 to 1500.

4. A method according to claim 1, characterized by a deasphaltation step performed on the sample before it is injected into the column.

5. An equipment for carrying-out the method according to one of the preceding claims, and comprising:

i) a injector for capillary columns;

ii) a gas chromatographic capillary column;

iii) an oven, wherein the column is housed, programmable in temperature;

iv) a detector of the flame ionization type, characterized in that said injector is of direct non vaporizing cold on-column type, that said capillary column has an inner diameter ranging between 0.5 and 0.6 mm and a length greater than 1 m and shorter than 10 m, with a thin-layer stationary phase in a thickness lower than or equal to 0.2 μm, and in that said oven is programmable over 400°C with a programmation rate equal to at least 5°C/min.

6. An equipment according to claim 5, characterized in that it comprises means for feeding carrier gas under conditions of constant pressure during the injection stage and of constant flow during the analysis stage.

7. An equipment according to claim 5, characterized in that said flame ionization detector comprises means for heating the body thereof at a temperature not lower than the maximum temperature programmed for the oven.

8. An equipment according to claim 5, characterized in that upstream said gas chromatographic column, a section of capillary column of about 1 m long, without stationary phase and deactivated, is provided for.

## Patentansprüche

1. Verfahren für die simulierte Destillation von Erdölprodukten, insbesondere von schweren Rückständen von Erdölprodukten, durch chromatographische Analyse und anschließende abschnittsweise Integration der Fläche, die zwischen der Elutionskurve der Probe und der Basislinie des Chromatogramms eingeschlossen ist, mit einer Umwandlung der Retentionszeiten in Siedetemperaturen mittels einer Standardmischung von dem Typ, der folgende Schritte umfaßt:

a) Vorsehen einer Anlage zur Gaschromatographenanalyse, umfassend:

i) einen Injektor für eine Kapillarsäule,

ii) eine Gaschromatographen-Kapillarsäule,

iii) einen Ofen, in welchem die Säule untergebracht ist und der hinsichtlich der Temperatur programmierbar ist,

iv) einen Detektor vom Flammenionisationstyp;

b) Injizieren einer in geeigneter Weise verdünnten Probe eines Erdölproduktes;

c) Durchführen einer Gaschromatographenanalyse mit einem Temperaturprogramm in dem Ofen;

d) Umwandeln der Zeitachse des erhaltenen Chromatogramms in die Siedepunktachse unter Verwendung von geeigneten Eichmischungen, die es ermöglichen, eine Eichkurve zu erhalten;

e) Integrieren in Abschnitten der Fläche zwischen der Probenelutionskurve und der Basislinie des Chromatogramms, um eine simulierte Destillations-Siedepunktverteilungskurve zu erhalten, dadurch gekennzeichnet, daß:

i) der Injector vom direkten, verdampfungsfreien kalten Typ direkt in der Säule ist;

ii) die Kapillarsäule einen Innendurchmesser im Bereich zwischen 0,5 und 0,6 mm und eine Länge hat, die größer als 1 m und kürzer als 10 m ist, mit einer stationären Dünnfilmphase, die kleiner oder gleich 0,2 µm ist, und daß das Temperaturprogramm in dem Ofen mit einer Programmiergeschwindigkeit durchgeführt wird, die mindestens gleich 5°C/min ist bis hinauf zu Temperaturen, die höher als 400°C sind.

2. Verfharen nach Anspruch 1, dadurch gekennzeichnet, daß die kalte Injektion und die Chromatographenanalyse durchgeführt werden durch die Zuführung des Trägergases bei einem konstanten Druck bzw. bei einer konstanten Strömung.

3. Verfahren nach Anspruch 1, wobei eine bekannte Substanz verwendet wird zur Definition der Eichkurve, bestehend aus einem Polyethylen POLYWAX® mit einem durchschnittlichen Molekulargewicht von 500 bis 1500.

4. Verfahren nach Anspruch 1, gekennzeichnet durch einen Entasphaltierungsschritt, der bei der Probe durchgeführt wird, bevor sie in die Säule injiziert wird.

5. Anlage zur Durchführung des Verfahrens nach einem der vorherigen Ansprüche und umfassend:

i) einen Injektor für Kapillarsäulen;

ii) eine Gaschromatographen-Kapillarsäule;

iii) einen Ofen, in welchem die Säule untergebracht ist und der hinsichtlich der Temperatur programmierbar ist;

iv) einen Detektor vom Flammenionisationstyp, dadurch gekennzeichnet, daß der Injektor vom direkten, verdampfungsfreien Typ direkt in der Säule ist, daß die Kapillarsäule einen Innendurchmesser im Bereich zwischen 0,5 und 0,6 mm und eine Länge hat, die größer als 1 m und kürzer als 10 m ist, mit einer stationären Dünnfilmphase in einer Dicke die kleiner oder gleich 0,2 µm ist, und

daß der Ofen über 400°C programmierbar ist mit einer Programmierrate, die mindestens gleich 5°C/min ist.

6. Anlage nach Anspruch 5, dadurch gekennzeichnet, daß sie eine Einrichtung für die Zuführung von Trägergas unter den Bedingungen von konstantem Druck während des Injektionsstadiums und von einer konstanten Strömung während des Analysestadiums aufweist.

7. Anlage nach Anspruch 5, dadurch gekennzeichnet, daß der Flammenionisationsdetektor eine Einrichtung aufweist, um seinen Körper auf eine Temperatur aufzuheizen, die nicht niedriger als die maximale Temperatur ist, für die der Ofen programmiert ist.

8. Anlage nach Anspruch 5, dadurch gekennzeichnet, daß stromaufwärts von der Gaschromatographensäule ein Abschnitt der Kapillarsäule von etwa 1 m Länge, ohne stationäre Phase und entaktiviert, vorgesehen ist.

**Revendications**

1. Procédé pour la distillation simulée de produits pétroliers, en particulier de résidus lourds de pétrole, au moyen de l'analyse chromatographique et de l'intégration par tranches consécutive de la surface comprise entre la courbe d'élution de l'échantillon et la ligne de base du chromatogramme, avec conversion des temps de rétention en températures d'ébullition au moyen d'un mélange étalon, du type comprenant les étapes suivantes:

a) l'aménagement d'un appareillage d'analyse chromatographique gazeuse, comprenant:

i) un injecteur pour colonne capillaire;

ii) une colonne capillaire pour chromatographie gazeuse;

iii) un four, dans lequel la colonne est logée, programmable en température;

iv) un détecteur du type à ionisation de flamme;

b) l'injecton d'un échantillon convenablement dilué d'un produit pétrolier;

c) l'exécution d'une analyse chromatographique gazeuse avec un programme de températures dans le four;

d) la conversion de l'axe des temps du chromatogramme obtenu en un axe des points d'ébullition en utilisant des mélanges d'étalonnage convenables qui permettent l'obtention d'une courbe d'étalonnage;

e) l'intégration par tranches de la surface comprise entre la courbe d'élution de l'échantillon et la ligne de base du chromatogramme pour obtenir une courbe de distribution des points d'ébullition de distillation simulée, caractérisé en ce que:

i) ledit injecteur est du type direct sur colonne à froid non vaporisant;

ii) ladite colonne capillaire a un diamètre intérieur s'étendant entre 0,5 et 0,6 mm et une longueur supérieure à 1 m et inférieure à 10 m, avec une phase stationnaire en film mince, inférieure ou égale à 0,2 µm, et en ce que ledit programme de températures dans le four est mis en oeuvre avec une vitesse de programmation égale au moins à 5°C/minute, jusquà des températures supérieures à 400°C.

2. Procédé selon la revendication 1, caractérisé en ce que ladite injection à froid et ladite analyse chromatographique sont exécutées en alimentant le gaz entraîneur de manière respective sous une pression constante et à un débit constant.

3. Procédé selon la revendication 1, dans lequel on utilise pour la définition de ladite courbe d'étalonnage une substance connue consistant en un polyéthylène POLYWAX® ayant un poids moléculaire moyen de 500 à 1500.

4. Procédé selon la revendication 1, caractérisé par une étape de désasphaltation exécutée sur l'échantillon avant de l'injecter dans la colonne.

5. Appareillage pour la mise en oeuvre du procédé selon l'une des revendications précédentes, et comprenant:

i) un injecteur pour colonnes;

ii) une colonne capillaire pour chromatographie gazeuse;

iii) un four, dans lequel la colonne est logée, programmable en température;

iv) un détecteur du type à ionisation de flamme, caractérisé en ce que ledit injecteur est du type direct sur colonne à froid non vaporisant, que ladite colonne capillaire a un diamètre intérieur s'étendant entre 0,5 et 0,6 mm et une longueur supérieure à 1 m et inférieure à 10 m, avec une phase stationnaire en couche mince en une épaisseur inférieure ou égale à 0,2 μm, et en ce que ledit four est programmable au-dessus de 400°C avec une vitesse de programmation égale au moins à 5°C/minute.

6. Appareillage selon la revendication 5, caractérisé en ce qu'il comprend un moyen pour alimenter le gaz entraîneur dans des conditions de pression constante pendant l'étape d'injection et de débit constant pendant l'étape d'analyse.

7. Appareillage selon la revendication 5, caractérisé en ce que ledit détecteur à ionisation de flamme comprend un moyen pour chauffer le corps de celui-ci à une température non inférieure à la température maximale programmée pour le four.

8. Appareillage selon la revendication 5, caractérisé en ce qu'en amont de ladite colonne chromatographique gazeuse est prévue une section de colonne capillaire d'une longueur d'environ 1 m, sans phase stationnaire et désactivée.

EP 0 186 845 B1

## Fig.1

## Fig.2

## Fig.3